# EUROPÄISCHE PATENTSCHRIFT

(11) **EP 0 489 049 B1**
(45) Veröffentlichungstag und Bekanntmachung des Hinweises auf die Patenterteilung: **07.12.1994**
(21) Anmeldenummer: 90912304.4
(22) Anmeldetag: 16.08.1990
(51) Int. Cl.: A61K 7/22

(54) **BELAGHEMMENDE ZAHNPASTE**
ANTI-PLAQUE TOOTHPASTE
DENTIFRICE CONTRE LA PLAQUE DENTAIRE

(30) Priorität: 25.08.1989 DE 3928063
(43) Veröffentlichungstag der Anmeldung: 10.06.1992
(73) Patentinhaber: Henkel Kommanditgesellschaft auf Aktien, 40191 Düsseldorf (DE)
(72) Erfinder: WÜLKNITZ, Peter, D-4018 Langenfeld-Berghausen (DE); LEHMANN, Rudolf, D-5653 Leichlingen 1 (DE); PLÖGER, Walter, D-4010 Hilden (DE); FÖRG, Franz, D-4018 Langenfeld (DE)
(86) Internationale Anmeldenummer: EP9001346
(87) Internationale Veröffentlichungsnummer: WO9102513

(56) Entgegenhaltungen:
- EP-A- 0 026 252
- EP-A- 0 304 627
- DE-A- 2 126 539

## Beschreibung

Die Erfindung betrifft eine Zahnpaste in Form einer Dispersion wasserunlöslicher Poliermittel in einem wäßrigen Träger, die als plaquehemmende Komponente eine antimikrobiell wirksame Biguanidverbindung enthält, und deren übrige Komponenten nach Art und Menge so ausgewählt sind, daß trotz relativ niedriger Dosierung der antimikrobiell wirksamen Biguanidverbindungen eine optimale Hemmung der Zahnbelagsbildung erreicht wird.

Es ist seit langem bekannt, daß antimikrobiell wirksame Biguanidverbindungen eine die Bildung der Zahnbeläge verhindernde Wirkung haben. Diese Wirkung wird jedoch durch viele in Zahnpasten übliche Komponenten, insbesondere durch bestimmte Poliermittel, durch viele Bindemittel bzw. Konsistenzregler, oberflächenaktive Stoffe, ja sogar durch Süßungsmittel stark verringert oder ganz aufgehoben.

Es hat daher nicht an Versuchen gefehlt, solche Komponenten zu finden, welche die Wirkung der antimikrobiellen Biguanide nicht beeinträchtigen. So wird z. B. in DE-OS 21 58 149 vorgeschlagen, als Poliermittel α-Aluminiumoxid-trihydrat in einer bestimmten Partikelgröße einzusetzen. In DE-OS 34 44 958 wird andererseits offenbart, daß bestimmte Tenside die Wirkung eines antimikrobiellen Biguanids verstärken können. Es wurde nun festgestellt, daß auch dieser Effekt durch bestimmte Zahnpastenkomponenten, z. B. durch anionische Bindemittel bzw. Konsistenzregler, anionische Süßstoffe und bestimmte Lösungsvermittler, die zur Solubilisierung gegebenenfalls enthaltener Aromaöle erforderlich sind, stark verringert wird. Eine Zahnpaste, die auch noch bei niedriger Dosierung der antimikrobiell wirksamen Biguanidverbindungen eine hohe Biguanid-Verfügbarkeit und eine befriedigende Plaque-Hemmung aufweist, ist dem Stand der Technik nicht zu entnehmen. Es war Ziel der vorliegenden Erfindung, eine solche Zahnpaste zu schaffen.

Gegenstand der Erfindung ist eine Zahnpaste in Form einer Dispersion, enthaltend
10 - 60 Gew.-% Poliermittel,
2 - 20 Gew.-% Feuchthaltemittel,
0,5 - 50 Gew.-% wasserlösliche Konsistenzregler,
0,02 - 0,5 Gew.-% antimikrobielle Biguanide,
1 - 5 Gew.-% weiterer Zusatzstoffe aus der Gruppe der oberflächenaktiven Stoffe, der Aromaöle und Süßungsmittel, dadurch gekennzeichnet, daß
- als Poliermittel überwiegend α-Aluminiumoxid-trihydrat,
- als Konsistenzregler nichtionische Polysaccharidderivate,
- als oberflächenaktive Stoffe ein Alkylglykosid und gegebenenfalls ein nichtionogener Lösungsvermittler für das Aromaöl und
- als Süßungsmittel L-Aspartyl-L-phenylalanin-methylester enthalten sind.

α-Aluminiumoxid-trihydrat, Al(OH)₃, ist ein bekanntes Poliermittel für Zahnpasten. Bevorzugt eignet sich eine gemahlene Qualität, deren Teilchen überwiegend (mindestens 98 %) kleiner als 50 µm, mit einem Mittelwert von circa 1 - 10 µm sind.

Bevorzugt ist in der erfindungsgemäßen Zahnpaste eine Mischung aus α-Aluminiumoxid-trihydrat (A) und einer schwach calcinierten Tonerde (B) im Gewichtsverhältnis A : B = 100 : (1 - 15) in einer Menge von 30 - 60 Gew.-% enthalten, weil dadurch eine besonders hohe Polierwirkung ohne aufrauhende Nebenwirkung erzielt wird.

Die schwach calcinierte Tonerde hat bevorzugt einen Gehalt von circa 20 Gew.-% Gamma-Aluminiumoxid (γ-Al₂-O₃) und circa 80 Gew.-% Alpha-Aluminiumoxid (α-Al₂O₃), eine Agglomeratgröße unter 20 µm, eine mittlere Primärkristallgröße von 0,5 - 1,5 µm und ein Schüttgewicht von 500 - 600 g/l.

Geeignete schwach calcinierte Tonerden werden durch Calcination aus Aluminiumhydroxid hergestellt. Aluminiumhydroxid geht durch Calcination in das bei Temperaturen oberhalb 1 200 °C thermodynamisch stabile α.Al₂O₃ über. Die bei Temperaturen zwischen 400 und 1 000 °C auftretenden, thermodynamisch instabilen Al₂O₃-Formen bezeichnet man als Gamma-Formen (vgl. Ullmann, Encyclopädie der technischen Chemie, 4. Auflage (1974) Band 7, Seite 298). Durch Wahl der Temperatur und der Zeitdauer bei der Calcination kann man den Calcinationsgrad, d. h. die Umwandlung in das thermodynamisch stabile α-Al₂O₃ auf beliebige Höhe einstellen. Man erhält durch schwache Calcination eine Tonerde mit einem Gehalt an γ-Al₂O₃, der um so niedriger ist, je höher die Calcinationstemperatur und je länger die Calcinationsdauer gewählt wird. Schwach calcinierte Tonerden unterscheiden sich von reinem α-Al₂O₃ durch eine geringere Härte der Agglomerate, eine größere spezifische Oberfläche und größere Porenvolumina.

Aluminiumoxid-Poliermittel verschiedener Calcinationsgrade, Mahlfeinheit und Schüttgewichte sind im Handel erhältlich, z. B. die "Poliertonerden" der Firma Giulini-Chemie.

Als Feuchthaltemittel kommen Glycerin, Sorbit, Propylenglykol und Polyethylenglykole in Frage, bevorzugt werden Glycerin und/oder Sorbit verwendet.

Geeignete wasserlösliche Konsistenzregler sind die nichtionischen Polysaccharidderivate, z. B. Methyl-, Hydroxyethyl- und Hydroxypropylether von Cellulose, Stärke, Guar und Pflanzengummen. Bevorzugt werden Hydroxyethylcellulose und Methylhydroxypropylcellulose eingesetzt.

Als antimikrobiell wirksame Biguanidverbindung wird bevorzugt das aus GB-A-705 838 bekannte 1,1'-Hexamethylenbis-[5-(4-chlorphenyl)-biguanid] ("Chlorhexidin") in Form eines wasserlöslichen, physiologisch verträglichen Salzes, z. B. in Form des Acetats oder als Gluconat eingesetzt. Andere erfindungsgemäß geeignete antimikrobielle Biguanidverbindungen sind z. B. das 1,1'-Hexymethylen-bis-[5-(4-fluorphenyl)-biguanid] (Fluorhexidin) die aus GB-A-702 268 bekannten Polyhexamethylenbiguanidverbindungen des Typs Vantocil^{(R)} IB (ICI), ferner die aus US-A-2 684 924, US-A-2 990 425, US-A-3 468 898, US-A-4 022 834, US-A-4 053 636 und US-A-4 198 392 bekannten antimikrobiellen Biguanidverbindungen.

Als oberflächenaktive Stoffe werden in den erfindungsgemäßen Zahnpasten nichtionogene Tenside vom Typ der Alkylglykoside eingesetzt. Alkylglykoside, ihre Herstellung und ihre Verwendung als oberflächenaktive Stoffe sind beispielsweise aus US-A-3 839 318, US-A-3 707 535, US-A-3 547 828, DE-A-19 43 689, DE-A-20 36 472 und DE-A-30 01 064 sowie EP-A-77 167 bekannt. Ihre Herstellung erfolgt insbesondere durch Umsetzung von Glucose oder Oligosacchariden mit primären Alkoholen mit 8 - 16 C-Atomen. Bezüglich des Glykosidrestes gilt, daß sowohl Monoglykoside, bei denen ein cyclischer Zuckerrest glykosidisch an den Fettalkohol gebunden ist, als auch oligomere Glykoside mit einem Olygomerisationsgrad (OG) bis vorzugsweise 3 geeignet sind. Bevorzugt geeignete Alkylglykoside für die Herstellung der erfindungsgemäßen Zubereitungen sind solche mit 8 - 18 C-Atomen in der Alkylgruppe und einem mittleren Oligomerisierungsgrad des Glycosidrestes von 1 - 3. Der Oligomerisierungsgrad (OG) ist dabei ein statistischer Mittelwert, dem eine für solche technischen Produkte übliche Homologenverteilung zugrunde liegt. Diese Alkylglykoside sind in der erfindungsgemäßen Zahnpaste bevorzugt in Mengen von 0,025 - 2,5 Gew.-% enthalten.

Die erfindungsgemäße Zahnpaste kann durch Zugabe von Aromaölen und Süßungsmitteln in ihren organoleptischen Eigenschaften verbessert werden. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z. B. Menthol, Carvon , Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Zur Solubilisierung dieser meist wasserunlöslichen Aromaöle in der Zahnpaste ist erfindungsgemäß ein nichtionogener Lösungsvermittler erforderlich. Solche Lösungsvermittler gehören zur Gruppe der oberflächenaktiven Verbindungen. Ein weiterer Gegenstand der Erfindung ist daher eine erfindungsgemäße Zahnpaste, die 0,1 - 0,5 Gew.-% eines Aromaöls und 0,1 - 0,7 Gew.-% eines nichtionogenen Lösungsvermittlers, bevorzugt aus der Gruppe der oxethylierten Fettsäureglyceride, der oxethylierten Fettsäuresorbitanpartialester oder Fettsäurepartialester von Glycerin- oder Sorbitan-Oxethylaten enthält. Lösungsvermittler aus der Gruppe der oxethylierten Fettsäureglyceride umfassen vor allem Anlagerungsprodukte von 20 - 60 Mol Ethylenoxid an Mono- und Diglyceride von linearen Fettsäuren mit 12 - 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 - 60 Mol Ethylenoxid an Sorbitanmonoester und Sorbitandiester von Fettsäuren mit 12 - 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 - 60 Mol Ethylenoxid an 1 Mol Glycerin oder an 1 Mol Sorbit.

Die erfindungsgemäße Zahnpaste enthält bevorzugt als Lösungsvermittler für gegebenenfalls enthaltene Aromaöle Anlagerungsprodukte von 20 - 60 Mol Ethylenoxid an gehärtetes oder ungehärtetes Ricinusöl (d. h. an Oxystearinsäure- oder Ricinolsäure-triglycerid), an Glycerin-mono- und/oder -distearat oder an Sorbitanmono- und/oder -distearat.

Als Süßungsmittel eignen sich entweder natürliche Zucker, z. B. Sucrose, Maltose, Lactose und Fructose oder synthetische Süßstoffe, jedoch bevorzugt nichtionogene oder amphotere Stoffe. Bevorzugt geeignet als Süßungsmittel ist der L-Aspartyl-L-phenyl-alanin-methylester, im Handel unter der Warenbezeichnung Aspartame^{(R)}.

Es können auch weitere bekannte Zahnpastenzusätze in untergeordneten Mengen von insgesamt bis zu höchstens 3 Gew.-% zugegeben werden, soweit diese mit dem antimikrobiellen Biguanid verträglich sind und dessen Wirkung nicht beeinträchtigen. Solche Zusätze sind z. B.
- karieshemmende Stoffe wie Natriumfluorid oder Natriummonofluorphospat,
- Pigmente wie z. B. Titandioxid,
- Farbstoffe
- pH-Stellmittel zur Einstellung eines pH-Wertes von bevorzugt 6 - 8 und Puffersubstanzen, z. B. Citronensäure und deren Salze oder Phosphorsäure und deren Alkalisalze,
- wundheilende und entzündungshemmende Stoffe wie z. B. Allantoin, Harnstoff, Azulen bzw. Kamillenwirkstoffe.

Die folgenden Beispiele sollen den Gegenstand der Erfindung näher erläutern.

### Beispiele

| Zahnpaste | | | | | |
|---|---|---|---|---|---|
| (Zusammensetzung in Gew.-%) | 1 | 2 | 3 | 4 | 5 |
| Aluminiumoxid-trihydrat¹) | 45,0 | 45,0 | 35,0 | 35,0 | 54,0 |
| Tonerde, schwach calciniert²) | - | 1,0 | - | 1,0 | 1,0 |
| Glycerin | 10,0 | 10,0 | 15,0 | 15,0 | 5,0 |
| Sorbit (70 %ig) | - | - | - | - | 5,0 |
| Hydroxyethylcellulose³) | 1,5 | 1,5 | 2,0 | 2,0 | 3,0 |
| Methylhydroxypropylcellulose⁴) | 1,5 | 1,5 | 1,5 | 1,5 | - |
| Chlorhexidin-digluconat | 0,3 | 0,3 | 0,2 | 0,2 | 0,2 |
| Alkyl(C_{12/14})-glucosid (O G = 1,3) | 0,25 | 0,25 | 0,25 | 0,25 | 0,5 |
| HR 60⁵) | 0,4 | 0,4 | 0,1 | 0,1 | 0,2 |
| Pfefferminzöl | 0,4 | 0,4 | 0,1 | 0,1 | 0,2 |
| Aspartame^{(R)} | 0,2 | 0,2 | 0,1 | 0,1 | 0,1 |
| | ad | ad | ad | ad | ad |
| Wasser | 100 | 100 | 100 | 100 | 100 |

| | | | | | |
|---|---|---|---|---|---|
| ¹) Es wurde Hydrated Alumina, Grade C 333 der Fa. ALCOA Chem, Div. eingesetzt | | | | | |
| ²) Es wurde das Handelsprodukt Poliertonerde eingesetzt | | | | | |
| ³) Es wurde das Handelsprodukt Cellobond HEC 400 T von BP eingesetzt | | | | | |
| ⁴) Es wurde das Handelsprodukt Culminal MHPC 100 von Henkel eingesetzt | | | | | |
| ⁵) Anlagerungsprodukt von 60 Mol Ethylenoxid an gehärtetes Ricinusöl | | | | | |

## Patentansprüche

1. Zahnpaste, in Form einer wäßrigen Dispersion, enthaltend
10 - 60 Gew.-% Poliermittel,
2 - 20 Gew.-% Feuchthaltemittel,
0,5 - 5 Gew.-% wasserlösliche Konsistenzregler,
0,02 - 0,5 Gew.-% antimikrobielle Biguanide und
1 - 5 Gew.-% weiterer Zusatzstoffe aus der Gruppe der oberflächenaktiven Stoffe, der Aromaöle und Süßungsmittel, dadurch gekennzeichnet, daß
- als Poliermittel überwiegend α-Aluminiumoxid-trihydrat,
- als Konsistenzregler nichtionische Polysaccharidderivate,
- als oberflächenaktive Stoffe ein Alkylglykosid und gegebenenfalls ein nichtionogener Lösungsvermittler für das Aromaöl und
- als Süßungsmittel L-Aspartyl-L-phenylalanin-methylester enthalten sind.

2. Zahnpaste nach Patentanspruch 1, dadurch gekennzeichnet, daß als Poliermittel eine Mischung aus α-Aluminiumoxid-trihydrat (A) und einer schwach calcinierten Tonerde (B) im Gewichtsverhältnis A : B = 100 : (1 - 15) in einer Menge von 30 - 60 Gew.-% enthalten ist.

3. Zahnpaste nach Patentanspruch 1 oder 2, dadurch gekennzeichnet, daß als antimikrobielles Biguanid das 1,1'-Hexamethylen-bis-(4-chlorphenyl)-biguanid (Chlorhexidin) in Form eines wasserlöslichen Salzes enthalten ist.

4. Zahnpaste nach einem der Patentansprüche 1 - 3, dadurch gekennzeichnet, daß als Alkylglykoside solche mit 8 - 18 C-Atomen in der Alkylgruppe und einem mittleren Oligomerisationsgrad des Glycosidrestes von 1 - 3 in einer Menge von 0,025 bis 2,5 Gew.-% enthalten ist.

5. Zahnpaste nach einem der Patentansprüche 1 - 4, dadurch gekennzeichnet, daß 0,1 - 0,5 Gew.-% eines Aromaöls und 0,1 - 0,7 Gew.-% eines Lösungsvermittlers aus der Gruppe der oxethylierten Fettsäureglyceride, der oxethylierten Fettsäuresorbitanpartialester oder der Fettsäurepartialester von Glycerin- oder Sorbitan-Oxethylaten enthalten ist.

## Claims

1. A toothpaste in the form of an aqueous dispersion containing
10 to 60% by weight polishing agents,
2 to 20% by weight humectants,
0.5 to 5% by weight water-soluble consistency regulators,
0.02 to 0.5% by weight antimicrobial biguanides,
1 to 5% by weight other additives from the group consisting of surfactants, flavoring oils and sweeteners,
characterized in that it contains
- predominantly α-aluminium oxide trihydrate as polishing agent,
- nonionic polysaccharide derivatives as consistency regulators,
- an alkyl glycoside and optionally a nonionic solubilizer for the flavouring oil as surfactants and
- L-aspartyl-L-phenyl alanine methyl ester as sweetener.

2. A toothpaste as claimed in claim 1, characterized in that it contains a mixture of α-aluminium oxide trihydrate (A) and a weakly calcined alumina (B) in a ratio by weight of A to B of 100 : (1 - 15) in a quantity of 30 to 60% by weight as polishing agent.

3. A toothpaste as claimed in claim 1 or 2, characterized in that it contains 1,1'-hexamethylene bis-(4-chlorophenyl)-biguanide (chlorhexidine) in the form of a watersoluble salt as the antimicrobial biguanide.

4. A toothpaste as claimed in any of claims 1 to 3, characterized in that alkyl glycosides containing 8 to 18 carbon atoms in the alkyl group and having an average degree of oligomerization of the glycoside residue of 1 to 3 are present in a quantity of 0.025 to 2.5% by weight as the alkyl glycosides.

5. A toothpaste as claimed in any of claims 1 to 4, characterized in that it contains 0.1 to 0.5% by weight of a flavoring oil and 0.1 to 0.7% by weight of a solubilizer from the group consisting of ethoxylated fatty acid glycerides, ethoxylated fatty acid sorbitan partial esters or fatty acid partial esters of glycerol or sorbitan ethoxylates.

## Revendications

1. Dentifrice sous la forme d'une dispersion aqueuse, renfermant
10 à 60 % en poids d'agent polissant,
2 à 20 % en poids de préservateur d'humidité,
0,5 à 5 % en poids de régulateur de consistance soluble dans l'eau,
0,02 à 0,5 % en poids de biguanide antimicrobien et
1 à 5 % en poids d'autres additifs faisant partie du groupe constitué des substances tensioactives, des huiles aromatiques et des édulcorants,
caractérisé en ce qu'il contient
- comme agent polissant, surtout de l'oxyde d'alpha-aluminium trihydraté,
- comme régulateur de consistance des dérivés de polysaccharide non ioniques,
- comme substances tensioactives, un alkylglycoside et, éventuellement, un solubiliseur non ionique pour l'huile aromatique et
- comme édulcorant, du méthylester de L-aspartyl-L-phénylamine.

2. Dentifrice selon la revendication 1, caractérisé en ce qu'il renferme comme agent polissant, un mélange d'oxyde d'alpha-aluminium trihydraté (A) et d'une aluminine légèrement calcinée (B) dans un rapport pondéral A:B de 100: (1-15), dans une proportion de 30 à 60 % en poids.

3. Dentifrice selon la revendication 1 ou 2, caractérisé en ce qu'il contient comme biguanide antimicrobien le 1,1'-hexaméthylène-bis-4-(chlorophényl)-biguanide (chlorhexidine) sous la forme d'un sel soluble dans l'eau.

4. Dentifrice selon une des revendications 1 à 3, caractérisé en ce qu'il renferme comme alkylglycosides, ceux avec 8 à 18 atomes de C dans le groupe alkyle et un degré d'oligomérisation moyen du radical glycoside de 1 à 3, dans une proportion de 0,025 à 2,5 % en poids.

5. Dentifrice selon une des revendications 1 à 4, caractérisé en ce qu'il renferme 0,1 à 0,5 % en poids d'une huile aromatique et 0,1 à 0,7 % en poids d'un solvant faisant partie du groupe constitué des glycérides d'acides gras oxéthylés, des esters partiels de sorbitane d'acides gras oxéthylés ou des esters partiels d'acides gras d'oxéthylates de glycérine ou de sorbitane.
